# EUROPEAN PATENT SPECIFICATION

(11) **EP 1 912 638 B1**
(45) Date of publication and mention of the grant of the patent: **30.03.2016**
(21) Application number: 06776665.9
(22) Date of filing: 08.08.2006
(51) Int. Cl.: A61K 31/20, A61K 31/202, A61P 3/06, A61P 9/10, A61P 25/00

(54) **COMPOSITION OF N-3 FATTY ACIDS HAVING HIGH CONCENTRATION OF EPA AND/OR DHA AND CONTAINING N-6 FATTY ACIDS**
ZUSAMMENSETZUNG VON N-3-FETTSÄUREN MIT HOHER KONZENTRATION VON EPA UND/ODER DHA UND N-6-FETTSÄUREN
COMPOSITION D'ACIDES GRAS N-3 PRESENTANT UNE FORTE TENEUR EN EPA ET/OU DHA ET CONTENANT DES ACIDES GRAS N-6

(30) Priority: 10.08.2005 IT MI20051560
(43) Date of publication of application: 23.04.2008
(73) Proprietor: PRO APARTS - INVESTIMENTOS E CONSULTORIA LDA, Madeira (PT)
(72) Inventor: BRUZZESE, Tiberio, 20146 Milano (IT)
(74) Representative: Ripamonti, Enrico
(86) International application number: PCT/EP2006/007823
(87) International publication number: WO 2007/017240

(56) References cited:
- EP-A- 0 454 102
- EP-A- 1 396 533
- EP-A1- 0 599 576
- WO-A-00/44361
- WO-A-87/03899
- WO-A-2004/091603
- MELLOR J E ET AL: "OMEGA-3 FATTY ACID SUPPLEMENTATION IN SCHIZOPHRENIC PATIENTS" HUMAN PSYCHOPHARMACOLOGY. CLINICAL AND EXPERIMENTAL, JOHN WILEY & SONS LTD, vol. 11, no. 1, 1996, pages 39-46, XP000921351 ISSN: 0885-6222

## Description

The present invention refers to a composition of long-chain polyunsaturated fatty acids containing at least 80% by weight of acids of the n-3 series, represented by eicosapentaenoic acid (EPA, C20:5 n-3, all-cis) and/or docosahexaenoic acid (DHA, C22:6 n-3, all-cis), and at least 3% by weight of acids of the n-6 series, represented by the acid C20:4 n-6 (all-cis) and by the acid C22:5 n-6 (all-cis). The composition is further characterized in highly preferred form by a content of other C20, C21 and C22 n-3 acids, different from EPA and DHA, lower than 3% and by other preferred specifications, for the n-6 and n-3 components, as specified herein below.

All the above mentioned acids can be present in the composition in the form of free acids, of salts with bases acceptable for dietetic and pharmaceutical use, of C1-C3 alkyl esters, preferably the ethyl esters. The described composition is useful for dietetic and pharmaceutical treatments recognized as sensitive to EPA and/or DHA action, as well as to the action of the other components, as they too are specified herein below.

The beneficial effects of n-3 acids, essentially of EPA and DHA, are reported with increasing frequency in the literature.

The patent IT 1235879, subsequently mentioned and modified by US 5656667, claims the treatment or the prophylaxis of multiple risk factors for cardiovascular disturbances, with reduction of hypertension, hypertriglyceridemia, hypercholesterolemia, of the coagulation factor VII activity and of platelet aggregability. The claimed n-3 fatty acids composition includes 80% by weight of said substances, represented by at least 75% by weight of EPA and DHA, and at least 3% by weight of other n-3 C20, C21 and C22 acids.

The patent EP-B-0409903 describes a procedure to prepare mixtures with high concentration of EPA and DHA and/ or their esters, useful for the treatment of hyperlipemia and related pathologies, thrombosis, myocardial infarction, platelet aggregation, coagulation processes in the prevention of atherosclerosis, cerebral infarction, lesions and occlusions caused by vasomotor spasms, and several other pathologies.

The patent US 5760081 describes the treatment by infusional route with a composition containing EPA, to prevent the imminent ventricular fibrillation in subjects under infarction situations.

The patent application WO 00/48592 describes the use of a mixture of EPA and DHA ethyl esters having a concentration > 25% to prevent death, particularly "sudden death", in patients who have suffered previous infarct episodes.

The patent application EP 1310249 claims the use of EPA and/or DHA in the primary prevention of cardiac pathologies of coronary origin (thrombosis, myocardial infarction) and in those pathologies caused by disorders of conduction of the rhythm (arrhythmia, fibrillation), as well as in the pathologies of mechanical type, like decompensation and cardiac insufficiency, due to deficiencies of cardiac pump.

The EP 1157692 patent application describes a composition of fatty acids containing at least 80% by weight of EPA and DHA, wherein other n-3 C20, C21 and C22 acids constitute less than 3%, and claims their utility for production of a medicine for treatment of multiple risk factors for cardiovascular illnesses and other pathologies sensitive to the action of EPA and DHA.

In following times, beside the efficacy in heart-related pathologies, higher evidence of EPA and DHA activity has been achieved in the tumour illnesses (RA Karamali et al., J. Nat. Cancer Inst. 75, 457, 1984), rheumatoid arthritis, connective tissue disease and inflammation (RA Lewis e KF Austen, J. Clin. Invest. 73, 889, 1984), psoriasis, multiple sclerosis, in the several central nervous system pathologies, like epilepsy and depression, and the degenerative diseases as Alzheimer's disease, and several others.

It must be observed that the action of EPA and DHA in several of such pathologies probably displays by means of the most different mechanisms, by acting as unmodified molecules, or after inclusion into the phospholipidic pool, etc, but in some cases it is also possible that their activity can be mediated by same or different metabolites that resulted essential in the treatment of atherosclerotic and cardiovascular illnesses.

To this purpose, in fact, the mentioned patent application WO 00/48592 reports f.i. that EPA, being a precursor of prostacycline PGI3 and of tromboxane TxA3, exerts a preventing effect on platelet aggregation and on haematic thrombus formation, that can be ascribed to inhibition of cyclooxygenase (similar effect to that of aspirin) and/or to competition with arachidonic acid for this enzyme, with consequent reduction in the synthesis of PGE2 and TxA2, which are well known platelet aggregating agents.

Further DHA, which is the most important component of cerebral lipids and a component of the platelet cell, intervenes indirectly also in increasing platelet fluidity, thus playing an important role in antithrombotic activity.

Unfortunately, whichever is the pathology under treatment and whichever is the action mechanism specific for said pathology, as a result of the several variables conditioning blood haemostasis (coagulation factors, number of platelets, aggregating/ disaggregating prostanoids, etc.), it can happen that a prolonged pharmacological treatment with n-3 acids, and/or a treatment in high doses, coupled with other individual factors, may lead to an extension of the bleeding time, even till to clear haemorrhage episodes.

Already a first specific study on the matter (J Dyerberg and HO Bang, Lancet 2, 433, 1979) reported a bleeding time of 8.05 minutes in a group of Eskimos (platelet count: 171,000/ ml) receiving a regular intake of n-3 acids (fish oil) in the diet, as compared to 4.76 minutes in a control group of Danes (platelets: 232,000/ ml) poorly feed with n-3 acids.

Several other studies (f.i. BA Bradlow, Thrombosis and omega-3 fatty acids. Epidemiological and clinical aspects, in "Health effects of polyunsaturated fatty acids in seafoods.", New York, Academic Press, 1986, 111-133; SH Jr. Goodnight, The antithrombotic effects of fish oil., ibidem) have then confirmed that diet n-3 acids can lead, beside a decreased platelet aggregation, also to a prolongation of the bleeding time. This effect has been particularly observed in diabetic patients (RS Tirvis et al., Clin. Chim. Acta 164, 315, 1987).

It is anyway obvious that the risk of bleeding and haemorrhage can be consciously taken in the coronaropathic- atherosclerotic patient at risk of thrombosis or infarction, in that it is directly opposed to his specific pathology, while it is unacceptable in other heart diseases, like either those involving disturbances of rhythm and electrical conduction (arrhythmia, fibrillation), or those of mechanical origin related to failure of cardiac pump (heart decompensation), where the platelet disaggregation is not the first therapeutic choice.

As much it is quite unacceptable the risk of taking n-3 acids, in high concentration or dosage, or for prolonged times, when the therapeutic treatment is addressed to pathologies unrelated to heart diseases, and anyway in all cases in which the patient is particularly prone to haemorrhage episodes (pre- or post- surgery situation, gastric or duodenal ulcer, hepatic cirrhosis, defects of coagulation and blood count, post-traumatic situation, etc.). We have so found, to face this problem, that the addition of small amounts of n-6 acids to a composition of EPA and/or DHA, and its repeated administration to the experimental animals, is able to bring back to normal values the time of bleeding provoked by an induced trauma.

More precisely, in a typical experiment (see Test 1 below) a composition containing at least 80% by weight of ethyl esters of EPA and DHA, was added with at least 3% by weight of ethyl esters of acids of the n-6 series, typically of acid C20:4 n-6 and C22:5 n-6, and administered orally to mice at the dose of 100 mg/ kg for 14 days: an increase of only about 40% of the bleeding time, subsequent to an induced trauma, was so obtained in comparison to the animals treated with the starting composition partially free from n-6 components, which showed at their turn a bleeding time doubled (increase of about 100%) in comparison to the untreated animals.

Similarly, the administration of a composition containing 5% of the same n-6 esters (or 8% of total n-6 esters) increased the bleeding time by only 15% about (10% about, respectively).

### TEST 1

5 Groups of 10 male mice each, weight 25-33 g, have been treated by oral route for 14 days with saline solution (group 1, control), with 100 mg/ kg/ day of a composition of ethyl esters of EPA and DHA 85.2% (n-3 C20, C21 and C22 2.5%; n-6 C20:4 and C22:5 1.2%), obtained according to EP 1157692 (group 2, reference), and with 100 mg/ kg/ day of 3 compositions (E, C and D of Example 2, respectively), obtained according to the present procedure (groups 3, 4 and 5, treated). The animals have then been submitted to isoflurane inhalation to induce general anesthesia.

Using a suitable blade, we went on then to cut exactly 0.5 cm of the distal tip of the tail, and immediately soon after to insert the treated tail in a tube containing a saline solution buffered with a phosphate buffer and pre-heated and maintained at 37°C. At this time a stopclock has been started and, holding the tail gently near its base, the venous blood has been let to flow into the tube, by observing when the bleeding stopped, so as to stop the stopclock at the same time and to record the precise bleeding time.

Once the bleeding had stopped for 10-15 seconds, the animal has been returned to his cage, leaving the anesthesia to gradually reverse. Rarely bleeding may resume, what anyway is not included in any case in the bleeding time.

### Results: bleeding time (seconds)

| | |
|---|---|
| Group 1: | 56 (51-62) |
| Group 2: | 123 (115-132) (#) |
| Group 3: | 80 (72-85) (#) (# #) |
| Group 4: | 66 (56-70) (# #) |
| Group 5: | 64 (58-70) (# #) |

t Student test: (#) P<0.05 vs Group 1; (# #) P<0.05 vs Group 2.

Conclusion: the administration of a composition of EPA and DHA (group 2) prolongs in statistically significant way the bleeding time, which on the contrary is gradually reduced in the presence of increasing quantities of n-6 polyunsaturated components.

To better define the composition in object, we specify that the content of EPA and/or DHA is at least 80% by weight, preferably at least 85% and at least 90% in the order. A minimum content of at least 40% by weight of EPA and of at least 34% of DHA will be also preferred, while their ratio will be generally comprised between 2:1 and 1:2, preferably between 1.5:1 and 0.9:1.

In all cases EPA and/or DHA can be present in the form of free acids, or of salts with bases acceptable for dietetic and pharmaceutical use, or of C1- C3 alkyl esters.

Typical acceptable organic bases will be choline and ethanolamine, lysine and arginine; typical inorganic bases will be sodium and potassium hydroxide, and others. Among the alkyl esters, ethyl ester is highly preferred.

We further specify that the acids of the n-6 series, essentially represented by the acid C20:4 n-6 and by the acid C22:5 n-6, they too present in the above defined form of acids, salts or esters, will have a content of at least 3% by weight, preferably at least 5% by weight, or at least 5.5-8% by weight referred to the total content of the n-6 series acids. The ratio between C20:4 n-6 and C22:5 n-6 will be generally comprised between 10:1 and 1:10, preferably between 3:1 and 1:3, and meanly around 1.1. The content of acid C22:5 n-6 will constitute at least 1.2% by weight, preferably at least 2% by weight.

The mentioned patent application EP 1157692, while claiming a concentrated composition of EPA and DHA, carefully describes in addition that the long chained C20, C21 and C22 n-3 acids, other than EPA and DHA (always present in the compositions of EPA and DHA, in that they are already present in itself the starting raw material of production, i.e. in fish oil) have not ever been individually isolated and tested pharmacologically, and must be therefore considered to all purposes as true impurities and undesired by-products of EPA and DHA. For this reason, and to limit or avoid abnormal pharmaco-therapeutic answers, said patent application targeted, in total opposition to what claimed in patent IT 1235879, to limit their content to less than 3%.

We agree with this consideration, and specify therefore, in relation to the composition of the present invention, that it will be further characterized by a content of C20, C21 and C22 n-3 acids- different from EPA and DHA- preferably lower than 3% by weight, particularly lower than 1.5% by weight. In addition, still preferentially, also the acids C21:5 n-3, and/or C20:4 n-3 and/or C22:5 n-3 will be individually lower than 1%, while the ratio between C20:4 n-3 and C22:5 n-3 will be generally comprised between 10:1 and 1:10, preferably between 3:1 and 1:3. Even here, said acids will be present as free acids, salts or C1-C3 alkyl esters, as above defined.

The composition of the invention so defined, it can be substantially obtained by means of two different procedures.

According to the first procedure, which is not preferred, particular intermediates are obtained according to procedures described in literature, like f.i. US 5130061, IT 1235879, JP 02/25447, WO 89/11521, IT 1205043, EP 1157692, and others, by using oils of marine origin as starting materials (fish oils, etc.) or even, if preferred or necessary, vegetable oils (seed oils, etc.), algal oils, etc.: this intermediate materials are constituted by a composition of high concentration of EPA and/or DHA and of low concentration of other C20, C21 and C22 n-3 acids, and by another composition enriched of n-6 acids. These compositions are further purified and modified in their composition by means of molecular distillation, to be repeated more times if necessary, till the desired composition. Suitable materials can also be found on the market (Sigma Co., USA), at least those at higher purity. The mixing, in suitable ratios, of the various compositions will then follow, so as to reach the desired composition. In any phase of the procedure, each component will be modified according to known techniques, to give the required free acid, salt, or ester. This procedure anyway is not as a whole preferred, in that- often requiring careful and repeated steps of distillation- it results to be work-consuming and expensive.

A second procedure, while still adopting said methods of literature, as above mentioned, involves *viceversa* a more careful selection of starting oils, what is essential to the procedure results, and can therefore lead directly to the desired compositions without requiring any mixing of intermediate compositions.

The starting oils- usually fish oils- will be then severely selected, so as to have the maximum content of EPA and/or DHA, usually EPA around 12-18% and DHA around 8-12% (much higher concentrations can be reached only when just one of the two components is prevailing); to have the minimum content of other C20, C21 and C22 n-3 acids, preferably lower than 3% (possibly not higher than 1.5%) as a whole, and lower than 1.0% (possibly not higher than 0.5%) individually; and finally to have a relatively high content of n-6 acids, particularly C20:4 n-6 and C22:5 n-6, preferably higher than 2.5% (and possibly not higher than 6.0% in total), being C22:5 n-6 >1.2%.

The ratio between the components will be obviously respected, particularly between EPA and DHA (from 2:1 to 1:2), while no difficulty usually there will be in searching an acceptable ratio between the components C20:C22 n-3 and n-6, considering the foreseen wide ratio (from 10:1 to 1:10). In defining the compositions of raw materials, it has been kept into consideration that the content of the more unsaturated acids will be inclined to increase in some phases of the procedure (f.i. the concentration, see below) and can be partly modulated in other phases (f.i. molecular distillation).

It is not possible to give since the beginning clear indications for an optimal selection of the starting fish oil, it is required above all a continuous and severe analytical monitoring, carried out possibly on big material batches so as to obtain constant mean compositions. Other selection criteria can be totally erroneous, as a consequence of the extreme variability due to geographical factors, fishing season, feeding sources (phytoplankton and predatory species), migratory and reproductive cycles, activity of various enzymes (desaturases and elongases) which are present in the different fish species, and to other factors. Because of this variability, the indication of a fish oil according to his source is certainly inadequate and can reserve great surprises, while the strict analytical control will result indispensable. Principle indications point out as preferable the oils of fish caught in equatorial areas, or of fresh water fish, as well as of aquaculture fish instead of wild fish, but all this can not give any assurance, and the use decision will only derive from the analytical control.

Starting from selected oils as above indicated, one proceeds then according to said methods of literature, including anyway the precautions suitable to our specific purpose. The fish oil is therefore submitted to a hydrolytic procedure, f.i. in presence of potassium hydroxide and in hydro-alcoholic medium, maintaining conditions suitable to avoid any possible degradation (inert atmosphere, presence of anti-oxidants, careful heating), so obtaining the salts of fatty acids and from these the corresponding acids (and optionally the esters). Alternatively the fish oils are directly submitted to transesterification (instead of hydrolysis) in presence of an alkanol, preferably ethanol, and of an acid or preferably of a basic catalyst. The esters of fatty acids are so obtained (and from these optionally the corresponding acids and salts).

The mixture of esters of fatty acids (or of the acids themselves) is then submitted f.i. to reaction with urea in ethanol or in methanol, so obtaining an insoluble complex with saturated and less unsaturated components, which is filtered off, by recovering then from the solution a composition strongly enriched of polyunsaturated components like EPA and/or DHA. If the composition doesn't reach the desired concentration (EPA and/or DHA > 80%), it can be submitted to a new complexation with reduced quantities of urea.

The final step of preparation may simply involve a purification phase, f.i. by a chromatographic process or a simply percolation on silica, if the composition- by means of an optimal selection of the raw material- is already conform to the required specifications: in this case the purpose is only that of eliminating several foreign materials and degradation products which are present in the composition (oligomers and polymers, unsaponifiable material, pesticides, polluting agents, heavy metals, chlorinated solvents, etc.) or of bringing back to normal values the usual technological parameters (acid value, peroxide value, iodine value, etc.).

Otherwise, more frequently and preferably, the composition is submitted to molecular distillation (or similar procedures, f.i. fractional extraction with supercritical fluids), so also obtaining- through elimination of various fractions- a significant modification of the mixture composition, which will so result conform to the pre-fixed specifications for the various components. We have found that the distillation temperature, even under high vacuum, is essentially dependent on the molecular size, i.e. on the number of carbon atoms of the fatty acid, f.i. lower for compounds C18 and increasing in the order for C20 and C22, and inside any range of temperature, lower for the less unsaturated and higher in the order for the more unsaturated ones. The distillation phase will be then carried out according to the available composition and according to the required specifications of distillate.

The order here mentioned of the preparative steps is the preferred one, but can be modified according opportunity and necessity. Equally at any step, acids, salts and esters can be transformed each other, according to known methods. The number of urea treatments, and the selection of distillate fractions, will be done according to the analytical results and the suitable decisions taken according to the art.

The claimed composition can be used for the manufacture of a preparation for dietetic and/or pharmaceutical use. This last preparation can be administered both by parenteral route, and much preferably by oral route, but any other administration route can be practised, either preferably those which guaranty a high systemic absorption, and the topic applications. In any case the administration route by far preferred is the oral route, by means of pharmaceutical formulations that can be obtained with techniques and excipients typical of oily active ingredients, as the compositions in object, according to the instructions described f.i. in Remington's Pharmaceutical Sciences Handbook, Hack Publ. Co., N.Y., USA. The preferred formulation is that in soft capsules, preferably in soft gelatine capsules, but also hard capsules oil-proof and tablets on solid excipients, emulsions, granules in dispersing excipients, drops, syrups, etc., can be used. The fatty acids of the composition will be present preferably in the form of ethyl esters.

In the oral use, the unitary dose comprises generally 250-1500 mg of active composition, preferably 500-1000 mg, and the daily dose is usually 0.3-5.0 g or more, preferably 0.5-3.0 g.

The various pharmaceutical formulations, also useful for dietetic use and as alimentary integrators, can or must also contain- beside the composition of the invention and other substances or drugs having complementary and/or synergic activity- one or more vehicles acceptable for human use, as known in the art, like diluents, binders, stabilizers, surfactants, lubricants and similar. Highly desired is the presence of antioxidant agents like vitamin E (tocoferols), butyl-hydroxyanisole, butyl-hydroxytoluene, ascorbyl palmitate and ascorbic acid, and similar agents. Also acceptable or requested is the presence of preservatives, colouring matters, flavours, sweeteners, etc..

With reference to the pharmaceutical use of the preparation containing the composition of the invention, the known pathologies of the cardiovascular and cardio-circulatory systems will be included, and their risk factors (hypertriglyceridemia, hypercholesterolemia, atherosclerosis, etc.), including those pathologies of coronary (leading to infarction and sudden death) and of vascular origin in general (stroke, ischemia and cerebral infarct), the pathologies caused by defects of electrical conduction (arrhythmia, both atrial and ventricular, fibrillation) and those provoked by mechanical causes related to the cardiac pump (heart failure and decompensation), etc., both for the primary and the secondary prevention (before evident heart disease, post-infarction).

Also included will be the pathologies related to other organs and tissues, to metabolism, etc., in any manner sensitive to the action of EPA, DHA and the other constituents of the composition, and their metabolites, all eicosanoids included.

The sensitive pathologies are represented by the central nervous system diseases (epilepsy, depression, bipolar illness, etc.), even of degenerative type, as well as the autoimmune diseases, tumour diseases, arthritis, connective tissue diseases, Crohn disease, psoriasis, and several other illnesses shown in the literature, all that in analogy with the use of similar compositions.

Particular indication will be addressed to all those patients who are potentially or practically affected with bleeding problems, including the lighter forms like nasal epistaxis, till those at higher risk, as haemorrhages, internal haemorrhages, etc..

Are here included the subjects with active ulcer, liver cirrhosis, tumour disease, etc., as well as the victims of traumatic events, of surgery, etc. and those affected by problems of coagulation and platelet aggregation.

The following Examples are addressed to better illustrate the invention.

### EXAMPLE 1

It is used a fish oil, pool of different lots, having a content of EP A and DHA relatively high (about 26-27% in total), a relatively "high" content of C20-C22 n-6 acids, and a relatively "low" content of C20, C21 and C22 n-3 acids. The following phases of transesterification with ethanol, fractioned complexing with urea, and molecular distillation, all of them carried out under controlled experimental conditions, are described in the next Examples.

In the following Table 1, it is reported an exemplifying composition of the starting oil, limited to the n-3 and n-6, C20, C21 and C22 polyunsaturated constituents of our interest: all the saturated and monounsaturated components will be anyway completely removed by complexing with urea, while also the polyunsaturated acids with lower unsaturation degree will be strongly reduced.

During the distillation phase, it will be also substantially reduced or eliminated the presence of all residual low-boiling acids till C18, with the only more evident exception of C18:4 n-3 and other components present in very low concentrations, usually < 0.2%.

In the same Table it is also reported, for the same acids, the composition of the obtained ethyl esters, which will result to be for EPA and DHA >80% (86.7%), ratio between 2 and 0.5 (1.25), for C20:4 and C22:5 n-6 >3.0% (5.5%), for the other C20, C21 and C22 n-3 <3.0% (1.7%).

**Table 1**

| Fatty acids | Composition in fatty acids of starting oil (%) | Composition of obtained ethyl esters (%) |
|---|---|---|
| C20:2 n-6 | 0.2 | 0.1 |
| C20:3 n-6 | 0.1 | 0.1 |
| C20:4 n-6 | 1.4 | 2.4 |
| C20:4 n-3 | 0.5 | 0.4 |
| C20:5 n-3 (EPA) | 16.8 | 48.2 |
| C21:5 n-3 | 0.4 | 0.6 |
| C22:2 n-6 | 0.6 | 0.1 |
| C22:4 n-6 | 0.4 | 0.6 |
| C22:5 n-6 | 2.3 | 3.1 |
| C22:5 n-3 | 0.5 | 0.7 |
| C22:6 n-3 (DHA) | 9.7 | 38.5 |
| Sum EPA + DHA | 26.5 | 86.7 |
| Ratio EPA:DHA | 1.73 | 1.25 |
| Sum n-6: C20:4+C22:5 | 3.7 | 5.5 |
| Sum n-6: total | 5.3 | 7.2 |
| Sum other n-3: C20, C21 and C22 | 1.4 | 1.7 |

### EXAMPLE 2

Some exemplifying compositions obtained according to the invention are reported.

The basic procedures are deduced from the literature, f.i. US 5130061, IT 1235879, JP 02/25447, WO 89/11521, EP 1310249, and others, taking into consideration the modifications and indications of the present Description, of Example 1, and of the following Examples. The compositions may contain d,1- alpha- tocopherol (>0.03%%) as antioxidant.

**Table 2**

| Compositions (%) | A1 | B1 | C1 | D1 | E1 | F1 | G1 | H2 | I3 | J1 | K1 |
|---|---|---|---|---|---|---|---|---|---|---|---|
| EPA | 46.3 | 55.4 | 46.2 | 41.5 | 49.8 | 37.9 | 51.2 | 45.8 | 45.5 | 81.7 | |
| DHA | 35.4 | 28.4 | 39.1 | 45.0 | 36.9 | 50.4 | 40.0 | 39.8 | 35.8 | | 80.6 |
| C20:4 n-6 | 2.5 | 3.0 | 3.0 | 2.6 | 2.5 | 1.7 | 0.8 | 0.4 | 1.8 | | |
| C22:5 n-6 | 4.3 | 3.2 | 2.8 | 5.5 | 1.2 | 3.6 | 3.5 | 3.0 | 4.2 | | |
| C20:4 n-3 | 0.6 | 0.8 | 0.4 | 0.1 | 0.5 | 0.9 | 0.3 | 0.9 | 0.2 | | |
| C21:5 n-3 | 0.9 | 0.8 | 0.6 | 0.5 | 0.7 | 0.8 | 0.5 | 0.6 | 0.9 | | |
| C22:5 n-3 | 1.4 | 0.9 | 0.9 | 0.7 | 0.6 | 0.9 | 0.7 | 0.1 | 0.8 | | |
| Sum EPA+DHA | 81.7 | 83.8 | 85.3 | 86.5 | 86.7 | 88.3 | 91.2 | 85.6 | 81.3 | | |
| Ratio EPA:DHA | 1.31 | 1.95 | 1.18 | 0.92 | 1.35 | 0.75 | 1.28 | 1.15 | 1.27 | | |
| Sum n-6 : C20:4+C22:5 | 6.8 | 6.2 | 5.8 | 8.1 | 3.7 | 5.3 | 4.3 | 3.4 | 6.0 | 8.1 | 8.9 |
| Sum n-6 total | 9.3 | 9.5 | 7.1 | 8.8 | 3.9 | 6.5 | 5.5 | 6.2 | 9.7 | | |
| Sum other n-3: C20, C21 and C22 | 2.9 | 2.5 | 1.9 | 1.3 | 1.8 | 2.6 | 1.5 | 1.6 | 1.9 | 2.7 | 2.6 |

| | | | | | | | | | | | |
|---|---|---|---|---|---|---|---|---|---|---|---|
| (1) Ethyl esters; (2) Free acids; (3) Sodium salts | | | | | | | | | | | |

### EXAMPLE 3

### a) Transesterification

It has been used a fish oil of composition similar to that reported in Example 1, characterized by a content of EPA and DHA (17.3% and 10.4%, respectively) and acids n-6 C20:4 and C22:5 (0.6% and 3.2%, respectively) relatively high, and by a content of other acids n-3 C20, C21 and C22 (1.5%, total), relatively low.

1.0 kg of oil is treated with 2 litres of ethanol and 12.5 g of potassium hydroxide, under stirring for 2 hours in nitrogen atmosphere, so obtaining a complete transformation of glycerides into ethyl esters.

The obtained solution is diluted with water, acidified with sulphuric acid and diluted with a double volume of hexane. The water- alcohol phase is then eliminated, while the organic phase is carefully washed with water, having care not to give emulsions, dried and evaporated to dryness under vacuum.

### b Complexing with urea

The mixture of ethyl esters obtained according to a), is treated with a boiling solution of 2.5 kg of urea in 8 litres of methanol, then the mixture is cooled to 0/+4°C, and left to stand overnight, obtaining an abundant precipitate constituted by the complex of urea with the esters of saturated and less unsaturated acids. The precipitate is filtered off and the methanol solution is distilled under vacuum till to oily residue. The residue is dissolved again with hexane, the solution is washed with water, dried and evaporated to dryness, obtaining a concentrate mixture of ethyl esters of polyunsaturated acids.

The mixture can be submitted then to new complexation with urea till to constant composition.

### c) Molecular distillation

The concentrated mixture of ethyl esters of polyunsaturated fatty acids is submitted to double stage molecular distillation, collecting the main fraction at 94-98 °C/
10-3 mm Hg. The composition, similarly to that obtained in Example 1), includes in the form of ethyl esters: EPA 47.7% and DHA 38.1%, C20:4 n-6 0.8%, C22:5
n-6 3.1%, other acids n-3 C20, C21 and C22 equal to 1.6%. If proper, the obtained mixture is submitted to new distillation (or purification by other route), according to the teaching of the present Description.

### EXAMPLE 4

The composition and the preparation of 1 g soft gelatine capsules is reported

| Composition | EPA and/or DHA (1) | d,l-α-tocoferol | Gelatine | Gelatine succinate | Glycerol | IO (2) | SEHB (3) | SPHB (4) |
|---|---|---|---|---|---|---|---|---|
| mg | 1000 | 0.3 | | 233 | 67 | | 0.09 | 0.54 |
| mg | 1000 | 4 UI | 246 | | 118 | 3.54 | | |

| | | | | | | | | |
|---|---|---|---|---|---|---|---|---|
| (1) Compositions as described in Examples 1 and 2; (2) Iron oxide; (3) Sodium ethyl p-hydroxybenzoate; (4) Sodium propyl p-hydroxybenzoate. | | | | | | | | |

Preparation: The composition of fatty acids according to Examples 1 and 2 and the excipients are weighed and homogenized in a tank with a high speed stirrer. The mixture is then treated with a colloidal mill and de-aerated in a stainless steel container. Proceed then to the inclusion in soft gelatine capsules by adopting standard methods and equipments.

## Claims

1. Composition of long chain polyunsaturated fatty acids containing at least 80% by weight eicosapentaenoic acid (EPA, C20:5 n-3) and/or docosahexaenoic acid (DHA, C22:6 n-3), and at least 3% by weight being the sum of C20:4 n-6 acid and the C22:5 n-6 acid, wherein the acids may be present in the form of free acids, or their salts with bases acceptable for dietetic and pharmaceutical use, or by C1-C3 alkyl esters.

2. Composition according to claim 1, wherein other n-3 acids C20, C21 and C22, different from EPA and DHA, constitute less than 3% by weight, particularly less than 1.5% by weight.

3. Compositions according to claims 1 and 2, wherein the acid C21:5 n-3 constitutes less than 1%.

4. Composition according to any of the preceding claims, wherein the acid C20:4 n-3 constitutes less than 1%.

5. Composition according to any of the preceding claims, wherein the acid C22:5 n-3 constitutes less than 1 %.

6. Composition according to any of the preceding claims, containing at least 85% by weight of EPA and/or DHA.

7. Composition according to any of the preceding claims, containing at least 90% by weight of EPA and/or DHA.

8. Composition according to any of the preceding claims, containing at least 5% by weight of C20:4 n-6 and C22:5 n-6.

9. Composition according to any of the preceding claims, containing at least 5.5% by weight of acids of n-6 series.

10. Composition according to any of the preceding claims, containing at least 8% by weight of acids of n-6 series.

11. Composition according to any of the preceding claims, containing at least 40% by weight of EPA.

12. Composition according to any of the preceding claims, containing at least 34% by weight of DHA

13. Composition according to any of the preceding claims, wherein the acid C22:5 n-6 constitutes at least 1.2%.

14. Composition according to any of the preceding claims, wherein the acid C22:5 n-6 constitutes at least 2%.

15. Composition according to any of the preceding claims, wherein the ratio between EPA and DHA is comprised between 2:1 and 1:2, preferably between 1.5:1 and 0.9:1.

16. Composition according to any of the preceding claims, wherein the ratio between the acid C20:4 n-6 and the acid C22:5 n-6 is comprised between 10:1 and 1:10, preferably between 3:1 and 1:3.

17. Composition according to any of the preceding claims, wherein the ratio between the acid C20:4 n-3 and the acid C22:5 n-3 is comprised between 10:1 and 1:10, preferably between 3:1 and 1:3.

18. Composition according to any of the preceding claims, wherein the salts with bases acceptable for dietetic and pharmaceutical use, are represented by salts with inorganic and organic bases.

19. Composition according to any of the preceding claims, wherein the C1-C3 alkyl esters are represented by ethyl esters.

20. Use of a composition according to claims 1-19, for the production of a dietetic preparation or a pharmaceutical preparation for treatment and/or prophylaxis of subjects affected by multiple risk factors for cardiovascular diseases, by cardiovascular and cardio-circulatory diseases, and by other pathologies sensitive to the action of EPA and/or DHA.

21. Use according to claim 20, wherein the multiple risk factors for cardiovascular diseases are represented by hypertriglyceridemia, hypercholesterolemia, hypertension and hyperactivity of factor VII of coagulation.

22. Use according to claim 20, wherein the cardiovascular and cardio-circulatory diseases derive from coronary and vascular problems, like atherosclerosis and pre- and post- infarct situations and like stroke, ischemia and brain infarction; from defects of electric conduction and rhythm, like tachycardia, atrial and ventricular arrhythmia, fibrillation and sudden death; from mechanical defects of heart pump, like heart failure and decompensation.

23. Use according to claim 20, wherein the sensitive pathologies are represented by the central nervous system diseases, like epilepsy, depression and bipolar forms, degenerative nervous illnesses, autoimmune pathologies, tumour diseases, arthritis and connective tissue diseases, Crohn disease, psoriasis, and others.

24. Use according to any of claims 20-23, wherein the subjects are easily affected by haemorrhage processes, as those affected by active ulcer, liver cirrhosis, tumour diseases, as well as those victims of traumatic and surgical events, and those affected by problems of coagulation and platelet aggregation.

25. A dietetic or pharmaceutical preparation comprising a composition according to any of claims 1-19.

26. A dietetic or pharmaceutical preparation according to claim 25, constituted by soft gelatine capsules.

27. A dietetic or pharmaceutical preparation according to claim 25 or 26, comprising a vehicle and/or excipient and/or diluent pharmacologically acceptable.

## Patentansprüche

1. Zusammensetzung aus langkettigen mehrfach ungesättigten Fettsäuren, enthaltend mindestens 80 Gew.-% Eicosapentaensäure (EPA, C20:5 n-3) und/oder Docosahexaensäure (DHA, C22:6 n-3), und mindestens 3 Gew.-% als Summe von C20:4 n-6-Säure und C22:5 n-6-Säure, wobei die Säuren in der Form freier Säuren, oder ihrer Salze mit Basen, die für diätetische und pharmazeutische Verwendung annehmbar sind, oder als C₁-C₃-Alkylester vorliegen können.

2. Zusammensetzung gemäß Anspruch 1, wobei andere n-3-Säuren C20, C21 und C22 als EPA und DHA weniger als 3 Gew.-%, insbesondere weniger als 1,5 Gew.-%, ausmachen.

3. Zusammensetzungen gemäß Anspruch 1 und 2, wobei die Säure C21:5 n-3 weniger als 1% ausmacht.

4. Zusammensetzung gemäß einem der vorangehenden Ansprüche, wobei die Säure C20:4 n-3 weniger als 1% ausmacht.

5. Zusammensetzung gemäß einem der vorangehenden Ansprüche, wobei die Säure C22:5 n-3 weniger als 1% ausmacht.

6. Zusammensetzung gemäß einem der vorangehenden Ansprüche, enthaltend mindestens 85 Gew.-% EPA und/oder DHA.

7. Zusammensetzung gemäß einem der vorangehenden Ansprüche, enthaltend mindestens 90 Gew.-% EPA und/oder DHA.

8. Zusammensetzung gemäß einem der vorangehenden Ansprüche, enthaltend mindestens 5 Gew.-% C20:4 n-6 und C22:5 n-6.

9. Zusammensetzung gemäß einem der vorangehenden Ansprüche, enthaltend mindestens 5,5 Gew.-% Säuren der n-6-Serie.

10. Zusammensetzung gemäß einem der vorangehenden Ansprüche, enthaltend mindestens 8 Gew.-% Säuren der n-6-Serie.

11. Zusammensetzung gemäß einem der vorangehenden Ansprüche, enthaltend mindestens 40 Gew.-% EPA.

12. Zusammensetzung gemäß einem der vorangehenden Ansprüche, enthaltend mindestens 34 Gew.-% DHA.

13. Zusammensetzung gemäß einem der vorangehenden Ansprüche, wobei die Säure C22:5 n-6 mindestens 1,2% ausmacht.

14. Zusammensetzung gemäß einem der vorangehenden Ansprüche, wobei die Säure C22:5 n-6 mindestens 2% ausmacht.

15. Zusammensetzung gemäß einem der vorangehenden Ansprüche, wobei das Verhältnis zwischen EPA und DHA zwischen 2:1 und 1:2, bevorzugt zwischen 1,5:1 und 0,9:1 liegt.

16. Zusammensetzung gemäß einem der vorangehenden Ansprüche, wobei das Verhältnis zwischen der Säure C20:4 n-6 und der Säure C22:5 n-6 zwischen 10:1 und 1:10, bevorzugt zwischen 3:1 und 1:3 liegt.

17. Zusammensetzung gemäß einem der vorangehenden Ansprüche, wobei das Verhältnis zwischen der Säure C20:4 n-3 und der Säure C22:5 n-3 zwischen 10:1 und 1:10, bevorzugt zwischen 3:1 und 1:3 liegt.

18. Zusammensetzung gemäß einem der vorangehenden Ansprüche, wobei die Salze mit Basen, die für diätetische und pharmazeutische Verwendung annehmbar sind, durch Salze mit anorganischen und organischen Basen dargestellt sind.

19. Zusammensetzung gemäß einem der vorangehenden Ansprüche, wobei die C1-C3-Alkylester durch Ethylester dargestellt sind.

20. Verwendung einer Zusammensetzung gemäß den Ansprüchen 1 bis 19 für die Herstellung eines diätetischen Präparats oder eines pharmazeutischen Präparats für die Behandlung und/oder Prophylaxe von Subjekten, welche von multiplen Risikofaktoren für kardiovaskuläre Krankheiten, von kardiovaskulären und Herz-Kreislauf-Krankheiten, und von anderen Pathologien, die gegenüber der Wirkung von EPA und/oder DHA empfindlich sind, betroffen sind.

21. Verwendung gemäß Anspruch 20, wobei die multiplen Risikofaktoren für kardiovaskuläre Krankheiten durch Hypertriglyzeridämie, Hypercholesterinämie, Hypertonie und Hyperaktivität von Gerinnungsfaktor VII dargestellt werden.

22. Verwendung gemäß Anspruch 20, wobei die kardiovaskulären und Herz-Kreislauf-Krankheiten von koronaren und vaskulären Problemen, wie Atherosklerose und Prä- und Post-Infarktsituationen und wie Schlaganfall, Ischämie und Hirninfarkt; von Defekten der elektrischen Leitung und des Rhythmus, wie Tachykardie, atriale und ventrikuläre Arrhythmie, Fibrillierung und plötzlichem Tod; von mechanischen Defekten der Herzpumpe, wie Herzversagen und Dekompensierung, herrühren.

23. Verwendung gemäß Anspruch 20, wobei die empfindlichen Pathologien durch die Krankheiten des Zentralen Nervensystems, wie Epilepsie, Depression und bipolare Formen, degenerative Nervenleiden, Autoimmunpathologien, Tumorkrankheiten, Arthritis und Krankheiten des Bindegewebes, Crohn-Krankheit, Psoriasis, und weiteren dargestellt werden.

24. Verwendung gemäß einem der Ansprüche 20 bis 23, wobei die Subjekte leicht von Hämorrhagieprozessen betroffen sind, wie diejenigen, die von aktiven Geschwüren, Leberzirrhose, Tumorkrankheiten betroffen sind, als auch diejenigen Opfer traumatischer und chirurgischer Ereignisse, und diejenigen, die von Problemen der Gerinnung und Blutplättchenaggregation betroffen sind.

25. Diätetisches oder pharmazeutisches Präparat, umfassend eine Zusammensetzung gemäß einem der Ansprüche 1 bis 19.

26. Diätetisches oder pharmazeutisches Präparat gemäß Anspruch 25, gebildet aus Weichgelatinekapseln.

27. Diätetisches oder pharmazeutisches Präparat gemäß Anspruch 25 oder 26, umfassend ein pharmakologisch annehmbares Vehikel und/oder Exzipiens und/oder Verdünnungsmittel.

## Revendications

1. Composition d'acides gras polyinsaturés à chaîne longue contenant au moins 80 % en poids d'acide eicosapentaénoïque (EPA, n-3 C20:5) et/ou d'acide docosahexaénoïque (DHA, n-3 C22:6), et au moins 3 % en poids constitués par la somme de l'acide n-6 C20:4 et de l'acide n-6 C22:5, dans laquelle les acides peuvent être présents sous la forme d'acides libres, ou de leurs sels avec des bases acceptables pour un usage diététique et pharmaceutique, ou par des esters d'alkyle C1-C3.

2. Composition selon la revendication 1, dans laquelle d'autres acides n-3 C20, C21 et C22, différents d'EPA et de DHA, constituent moins de 3 % en poids, en particulier moins de 1,5 % en poids.

3. Compositions selon les revendications 1 et 2, dans lesquelles l'acide n-3 C21:5 constitue moins de 1 %.

4. Composition selon l'une quelconque des revendications précédentes, dans laquelle l'acide n-3 C20:4 constitue moins de 1 %.

5. Composition selon l'une quelconque des revendications précédentes, dans laquelle l'acide n-3 C22:5 constitue moins de 1 %.

6. Composition selon l'une quelconque des revendications précédentes, contenant au moins 85 % en poids d'EPA et/ou de DHA.

7. Composition selon l'une quelconque des revendications précédentes, contenant au moins 90 % en poids d'EPA et/ou de DHA.

8. Composition selon l'une quelconque des revendications précédentes, contenant au moins 5 % en poids de n-6 C20:4 et de n-6 C22:5.

9. Composition selon l'une quelconque des revendications précédentes, contenant au moins 5,5 % en poids d'acides de la série des n-6.

10. Composition selon l'une quelconque des revendications précédentes, contenant au moins 8 % en poids d'acides de la série des n-6.

11. Composition selon l'une quelconque des revendications précédentes, contenant au moins 40 % en poids d'EPA.

12. Composition selon l'une quelconque des revendications précédentes, contenant au moins 34 % en poids de DHA.

13. Composition selon l'une quelconque des revendications précédentes, dans laquelle l'acide n-6 C22:5 constitue au moins 1,2 %.

14. Composition selon l'une quelconque des revendications précédentes, dans laquelle l'acide n-6 C22:5 constitue au moins 2 %.

15. Composition selon l'une quelconque des revendications précédentes, dans laquelle le rapport entre l'EPA et le DHA est compris entre 2:1 et 1:2, de préférence entre 1,5:1 et 0,9:1.

16. Composition selon l'une quelconque des revendications précédentes, dans laquelle le rapport entre l'acide n-6 C20:4 et l'acide n-6 C22:5 est compris entre 10:1 et 1:10, de préférence entre 3:1 et 1:3.

17. Composition selon l'une quelconque des revendications précédentes, dans laquelle le rapport entre l'acide n-3 C20:4 et l'acide n-3 C22:5 est compris entre 10:1 et 1:10, de préférence entre 3:1 et 1:3.

18. Composition selon l'une quelconque des revendications précédentes, dans laquelle les sels avec des bases acceptables pour un usage diététique et pharmaceutique, sont représentés par des sels avec des bases inorganiques et organiques.

19. Composition selon l'une quelconque des revendications précédentes, dans laquelle les esters d'alkyle C1-C3 sont représentés par des esters d'éthyle.

20. Utilisation d'une composition selon les revendications 1 à 19, pour la production d'une préparation diététique ou d'une préparation pharmaceutique destinée à traiter et/ou à prévenir les sujets affectés par de multiples facteurs de risque pour les maladies cardiovasculaires, par les maladies cardiovasculaires et cardio-circulatoires, et par d'autres pathologies sensibles à l'action de l'EPA et/ou du DHA.

21. Utilisation selon la revendication 20, dans laquelle les multiples facteurs de risque pour les maladies cardiovasculaires sont représentés par l'hypertriglycéridémie, l'hypercholestérolémie, l'hypertension et l'hyperactivité du facteur VII de coagulation.

22. Utilisation selon la revendication 20, dans laquelle les maladies cardiovasculaires et cardio-circulatoires dérivent de problèmes coronariens et vasculaires, comme l'athérosclérose et de situations pré- et post-infarctus, comme l'AVC, l'ischémie et l'infarctus cérébral ; de défauts de conduction électrique et de rythme, comme la tachycardie, l'arythmie atriale et ventriculaire, la fibrillation et la mort subite ; de défauts mécaniques de la pompe cardiaque, comme l'insuffisance cardiaque et la décompensation.

23. Utilisation selon la revendication 20, dans laquelle les pathologies sensibles sont représentées par les maladies du système nerveux central, comme l'épilepsie, la dépression et les formes bipolaires ; les maladies nerveuses dégénératives, les pathologies auto-immunes, les maladies tumorales, l'arthrite et les maladies du tissu conjonctif, la maladie de Crohn, le psoriasis, et autres.

24. Utilisation selon l'une quelconque des revendications 20 à 23, dans laquelle les sujets sont facilement affectés par des processus hémorragiques, tels que ceux affectés par un ulcère actif, la cirrhose du foie, des maladies tumorales, ainsi que ceux victimes d'événements traumatiques et chirurgicaux, et ceux affectés par des problèmes de coagulation et d'agrégation des plaquettes.

25. Préparation diététique ou pharmaceutique comprenant une composition selon l'une quelconque des revendications 1 à 19.

26. Préparation diététique ou pharmaceutique selon la revendication 25, constituée par des capsules en gélatine souples.

27. Préparation diététique ou pharmaceutique selon la revendication 25 ou 26, comprenant un véhicule et/ou un excipient et/ou un diluant pharmacologiquement acceptable.
